# EUROPÄISCHE PATENTANMELDUNG

(11) **EP 1 726 330 A2**
(43) Veröffentlichungstag der Anmeldung: **29.11.2006**
(21) Anmeldenummer: 06100259.8
(22) Anmeldetag: 12.01.2006
(51) Int. Cl.: A61Q 1/06, A61Q 1/08, A61K 8/49

(54) **Kosmetische Zubereitungen zur dekorativen Anwendung auf der Haut enthaltend Chinacridon-Pigmente**

(30) Priorität: 28.01.2005 DE 102005004179; 04.07.2005 DE 102005032238
(71) Anmelder: Beiersdorf AG, 20253 Hamburg (DE)
(72) Erfinder: Schaefer, Jessica, 22299, Hamburg (DE); Mocigemba, Nicole, 22529, Hamburg (DE); Lanzendoerfer, Ghita, 90491, Nürnberg (DE); Kallmayer, Volker, 22525, Hamburg (DE); Viala, Sophie, 20257 Hamburg (DE); Riedel, Heidi, 22083 Hamburg (DE)

(57) **Zusammenfassung**

Kosmetische Zubereitung zur dekorativen Anwendung auf der Haut, dadurch gekennzeichnet, dass sie Chinacridon-Pigmente und ein Öl, welches eine Grenzflächenspannung gegenüber Wasser kleiner als 30 mN/m aufweist, enthält.

## Beschreibung

Die Erfindung betrifft kosmetische Zubereitungen zur dekorativen Anwendung auf der Haut, die Chinacridon-Pigmente und eine Ölphase enthalten.

Chinacridone sind polycyclische Pigmente mit dem Grundkörper gemäß Formel I, wobei die Reste R¹ bis R⁸ gleich oder verschieden, Wasserstoffatome, Methylgruppen und Chloratome sein können. Sie sind als temperatur- und lichtstabile Pigmente für Lacke und Beschichtungen, für Drucktinten und zum Anfärben von thermoplastischen Kunststoffen bekannt.

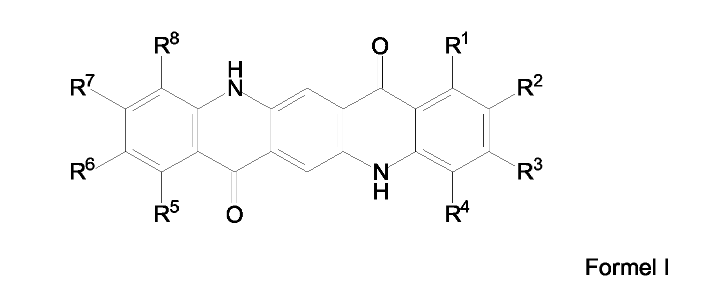

Chinacridone existieren in verschiedenen Kristall-Modifikationen, die durch verschiedene Nachbehandlungen aus dem Rohpigment erhalten werden. Zusätzlich lassen sich mit Mischkristallphasen ("Solid Solutions") Farbtöne erzielen, die mit einheitlichen Chinacridonphasen nicht zugänglich sind. Um zum einen die Bildung von Agglomeraten zu minimieren und zum anderen eine möglichst gleichmäßige Verteilung der Pigmentpartikel zu erreichen, sind je nach Einsatzbereich verschiedene Pigmentzubereitungen bekannt (siehe auch Heinrich Zollinger, Color Chemistry, Wiley VCH, Weinheim 2003, 321 ff).

So beschreibt US 6,063,182 eine Pigmentzusammensetzung zum Einrühren in wässrige Tinten und Anstrichfarben, die wasserlösliche Polyvinylpyrrolidon/Vinylacetat Copolymere enthält.

EP 1 031 615 beschreibt ein Verfahren zur Herstellung von linearen Chinacridonen mit verringerter Partikelgröße. Das erhaltene Pigment benötigt keine Nachbehandlung mehr und kann zum Einfärben von thermoplastischen Kunststoffen, Glas und keramischen Materialien verwendet werden. Es ist zur Pigmentierung von Tinten und insbesondere von wasser- oder lösemittelbasierten Beschichtungen und Anstrichfarben geeignet. Es kann auch in entsprechenden kosmetischen Formulierungen eingesetzt werden.

Die Verwendung von Chinacridon-Pigmenten in kosmetischen Zubereitungen, die eine für kosmetischen Anwendungen geeignete Ölphase und gegebenenfalls eine wässrige Phase enthalten, wird jedoch nicht beschrieben.

Aufgabe der vorliegenden Erfindung ist es daher, Zubereitungen, insbesondere zur dekorativen Anwendung auf der Haut, zur Verfügung zu stellen, die stabil sind und einen gleichmäßig deckenden Auftrag ermöglichen.

Die Aufgabe wird durch kosmetische Zubereitungen gelöst, die Chinacridon-Pigmente und ein oder mehrere Öle enthalten, wobei mindestens eines der Öle eine Grenzflächenspannung gegenüber Wasser kleiner als 30 mN/m hat.

Öle und Fette unterscheiden sich unter anderem in ihrer Polarität. Die Grenzflächenspannung gegenüber Wasser ist ein relevantes Maß für die Polarität eines Öles, insbesondere in Emulsionen. Die Polarität eines Öles ist umso größer, je niedriger die Grenzflächenspannung zwischen diesem Öl und Wasser ist. Erfindungsgemäß wird die Grenzflächenspannung als ein mögliches Maß für die Polarität einer Ölkomponente angesehen.
Die Grenzflächenspannung ist diejenige Kraft, welche an einer gedachten, in der Grenzfläche zwischen zwei Phasen befindlichen Linie der Länge von einem Meter wirkt. Die physikalische Einheit der Grenzflächenspannung ist demgemäß mN/m also Kraft/Strecke. Das Vorzeichen ist positiv, wenn die Grenzflächenspannung das Bestreben hat, die Grenzfläche zu verkleinern.

Um einen gleichmäßig deckenden Auftrag der Zubereitung auf die Haut zu erhalten, müssen die Pigmentpartikel in der Zubereitung möglichst fein und gleichmäßig dispergiert werden. Zu diesem Zweck werden üblicherweise Benetzungsmittel eingesetzt. Die Benetzungsmittel sollen auf die Oberfläche der Pigmentpartikel aufziehen und eine Schicht ausbilden, die sowohl die Agglomeratbildung der Partikel minimiert als auch die Verteilung der Partikel im Dispersionsmittel verbessert und stabilisiert. Die Eignung einer Substanz als Benetzungsmittel hängt damit sehr stark von den physikalisch-chemischen Eigenschaften des Systems, in welches das Pigment eingearbeitet werden soll, und vom Pigment selbst ab.

Es wurde überraschenderweise gefunden, dass Öle mit einer Grenzflächenspannung gegenüber Wasser kleiner 30 mN/m, bevorzugt kleiner 25 mN/m, besonders bevorzugt kleiner 20 mN/m als Benetzungsmittel für die Verteilung von Chinacridon-Pigmenten in kosmetischen Ölen, Wachsen und deren Mischungen sowie für Emulsionen aus wässrigen und öligen Phasen besonders gut geeignet sind.
Erfindungsgemäß bevorzugt weisen diese Öle zudem bei Raumtemperatur eine Viskosität auf, die größer als 2 mPas, bevorzugt größer als 10 mPas, mehr bevorzugt größer als 20 mPas, ist. Die Viskosität wird mit einem Gerät der Firma Rheometric Scientific (jetzt TA-Instruments) der Serie ARES mit der Messgeometrie Kegel/Platte bestimmt, wobei D = 50 mm, der Kegelwinkel = 0,02° und die Scherraten im Bereich zwischen 1 und 1000 s⁻¹ liegen.

Bevorzugte erfindungsgemäße Öle mit Grenzflächenspannung gegenüber Wasser kleiner als 30 mN/m sind aliphatische Kohlenwasserstoffe, die linear oder verzweigt und auch ungesättigt oder gesättigt sein können. Besonders bevorzugt werden die Öle aus der Gruppe enthaltend natürliche und synthetische Triglyceride wie z. B. Sojaöl, natürliche und synthetische Ester wie z. B. Jojobaöl, Methylpalmitat und Isopropylpalmitat, Ester aromatischer Carbonsäuren wie z. B. C₁₂₋₁₅-Alkylbenzoat, Alkohole und Ether wie z. B. Dicaprylylether sowie deren Mischungen gewählt.

Erfindungsgemäß ganz besonders bevorzugt sind Öle aus der Gruppe enthaltend Ricinusöl, Capryl-/Caprinsäuretriglyceride, Triisostearin, Cocoglyceride, Octyldodecanol, Isopropylstearat, Isononylisostearat, Isocetylstearoylstearat, Pentaerythrit-tri/tetraisostearat, Diisostearylmaleat, Trimethylolpropantriisostearat, Distearylfumarat, PPG-15-Stearylether, Lanolin, Hexadecan, Isohexadecan, Dodecan und Isododekan.

Kosmetische Zubereitungen, welche die Kombination von Chinacridon-Pigmenten mit dem erfindungsgemäßen Öl enthalten, zeichnen sich durch eine hervorragende Stabilität aus. So können erfindungsgemäß insbesondere auch Emulsionen pigmentiert werden, deren Stabilität ansonsten in der Regel durch die Zugabe von Feststoffen beeinträchtigt wird. Ferner wird bei Anwendung kosmetischer Zubereitungen im Sinne der vorliegenden Erfindung ein gleichmäßig deckender Farbauftrag erreicht.

Die erfindungsgemäßen Zubereitungen können Dispersionen von festen Chinacridon-Pigmenten in Ölen sein, welche zur Pigmentierung in eine kosmetische Zubereitung eingearbeitet wird. Diese Pigment-Dispersionen können in unterschiedlichen Viskositäten erhalten werden. Dabei variieren die Viskositäten von gut fließend über pastös bis fest. Die erfindungsgemäßen Pigment-Dispersionen lassen sich in ÖI/Wachs-basierte und ÖI/Wasser-basierte Systeme einarbeiten.

Die erfindungsgemäßen Pigment-Dispersionen enthalten 10 bis 80 Gew.-%, bevorzugt 20 bis 60 Gew.-%, besonders bevorzugt 30 bis 50 Gew.-%, Chinacridon-Pigmente, wobei die erfindungsgemäßen Endzubereitungen (z. B. Lippenstifte und Emulsionen) dann 0,01 bis 20 Gew.-%, bevorzugt 0,05 bis 15 Gew.-%, besonders bevorzugt 0,1 bis 12 Gew.-%, Chinacridon-Pigmente enthalten.

Zur Herstellung der Pigment-Dispersionen (Pigmentphase) werden die Chinacridon-Pigmente in dem erfindungsgemäßen Öl mit Hilfe eines Dreiwalzenstuhls, eines Dispermaten oder anderer gängiger Dispersions- und Mahlwerkzeuge dispergiert. Zusätzlich können weitere Bestandteile der Ölphase als Dispersionsmittel zugegeben werden.

Die Ölphase kann neben den erfindungsgemäßen Ölen mit einer Grenzflächenspannung gegenüber Wasser kleiner als 30 mN/m ferner eine oder mehrere der folgenden Substanzen enthalten:
● Mineralöle und andere Kohlenwasserstoffe wie z. B. Polydecene, Eicosan oder Isoeicosan,
● Ester aus organischen Säuren mit 3 bis 30 Kohlenstoffatomen, die verzweigt oder unverzweigt, gesättigt oder ungesättigt sein können, gegebenenfalls mit zusätzlichen Hydroxygruppen, und Alkoholen mit 2 bis 30 Kohlenstoffatomen, die 1 bis 5 Hydroxygruppen enthalten können und die verzweigt oder unverzweigt, gesättigt oder ungesättigt sein können, wobei die Säuren ganz oder teilweise verestert sind, wie z. B. Pentaerythrit-tri/tetraisostearat, Sheabutter, Cetylactat,
● Ether aus Alkoholen mit 2 bis 30 C-Atomen, die 1 bis 5 Hydroxygruppen enthalten können und die verzweigt oder unverzweigt, gesättigt oder ungesättigt sein können, wobei die Alkohole ganz oder teilweise verethert sein können,
● Ester der Kohlensäure mit Alkoholen mit 2 bis 30 C-Atomen, die 1 bis 5 Hydroxygruppen enthalten können und die verzweigt oder unverzweigt, gesättigt oder ungesättigt sein können, wie z. B. Dicaprylylcarbonat,
● cyclische oder lineare, verzweigte oder unverzweigte Silikonöle sowie
● Ester aromatischer Carbonsäuren mit einer Grenzflächenspannung gegenüber Wasser über 30 Nm/m.

Diese Substanzen können synthetisch oder natürlichen Ursprungs und gegebenenfalls raffiniert sein. Natürliche Öle, Fette und Wachse enthalten oft vorteilhafte Minderkomponenten wie Vitamine, Phytosterole und dergleichen. Besonders geeignete Substanzen für die Ölphase können bei Raumtemperatur flüssig, halbfest oder fest sein und scharfe Schmelzpunkte oder breite Schmelzbereiche aufweisen.

Die Zubereitungen im Sinne der vorliegenden Erfindung können fest, flüssig oder halbfest sein. Sie enthalten mindestens eine Phase (Pigmentphase), in welcher die Feststoffe, Pigmente und/oder Füllstoffe, dispergiert sind, und mindestens eine weitere Phase, die flüssig oder fest ist. Die weitere feste Phase kann als feste Bestandteile Wachse enthalten. Die weiteren flüssigen Phasen können eine oder mehrere Ölphasen und zusätzlich eine oder mehrere Wasserphasen enthalten. Die erfindungsgemäßen Zubereitungen können beispielsweise in Form von W/O-, O/W-, W/ONV- oder O/W/O-Emulsionen vorliegen. Darüber hinaus können solche Systeme zusätzlich in Öl- oder Wasserphasen dispergierte Feststoffe oder Gasblasen enthalten. Die erfindungsgemäßen Zubereitungen können in Form von Stiften, Schäumen (so genannten Mousse), Feststoff-Emulsionen (d. h. Emulsionen, welche durch Feststoffe stabilisiert sind wie z. B. Pickering-Emulsionen), sprühbaren Emulsionen oder Hydrodispersionen vorliegen.

Bevorzugte erfindungsgemäße Zubereitungen, welche als zusätzliche feste Phase Wachse enthalten (ÖI/Wachs-Systeme), sind beispielsweise Massen, die in Formen, insbesondere Stifte und Pfännchen gegossen werden, und Pasten.

Technisch betrachtet sind die erfindungsgemäßen Massen wasserfreie Fettmischungen aus festen oder halbfesten Wachsen und flüssigen Ölen. Das Verhältnis von Wachs zu Öl bestimmt die Härte der Masse und den Abrieb auf der Haut. Über die Änderung dieses Verhältnisses kann die Härte und Abrieb entsprechend eingestellt werden. So liegt z. B. für Lippenstifte das Verhältnis von Wachsen zu Ölen üblicherweise zwischen 10 Gew.-% zu 90 Gew.-% und 25 Gew.-% zu 75 Gew.-%.

Bevorzugte Grundstoffe für die erfindungsgemäßen Massen sind beispielsweise feste Bestandteile wie Bienenwachs, Ceresin oder mikrokristalline Wachse bzw. Ozokerit und hochschmelzende Wachse wie Carnaubawachs oder Candelillawachs, halbfeste Bestandteile wie Vaseline oder Lanolin und flüssige Öle wie Paraffinöle, Ricinusöl, Isopropylmyristat, C₁₂₋₁₅ Alkylbenzoat oder Diisostearylmaleat.

Zur Herstellung der erfindungsgemäßen Massen werden zunächst Chinacridon-Pigmenten in einem oder mehreren der erfindungsgemäßen polaren Ölen wie oben beschrieben dispergiert. Anschließend werden die restlichen Rezepturbestandteile bevorzugt unter Erwärmung auf Temperaturen zwischen 70 und 100°C aufgeschmolzen, vermischt und die Pigmentphase in die geschmolzene Mischung eingearbeitet. Die Masse wird anschließend in entsprechende Formen vergossen. Aufgrund der relativ hohen Temperaturen ist die erfindungsgemäße Masse oxidationsgefährdet, daher werden üblicherweise Antioxidantien zugesetzt und keine oxidationsempfindliche Inhaltsstoffe eingesetzt.

Bei der Herstellung von Lippenstiften werden die Stifte nach dem Erkalten aus der Form genommen und in Hülsen verbracht. Andere Zubereitungen wie z. B. gegossene Blushes verbleiben in der Form.

Erfindungsgemäße Zubereitungen, die neben der Pigmentphase Öl- und Wasserphasen (ÖI/Wasser-System) enthalten, sind beispielsweise Emulsionen oder Hydrodispersionen. Emulsionen im Sinne der Erfindung können O/W-, W/O-, W/Si- (Wasser-in-Silikonöl), O/W/O-, W/O/W- oder Feststoff-Emulsionen sein. Diese Emulsionen können auch fest, sprühbar oder fließfähig sein. Erfindungsgemäße Emulsionen enthalten neben der Ölphase, die aus Ölen, Fetten, Wachsen oder deren Mischungen bestehen kann, Wasser und vor allem Emulgatoren sowie gegebenenfalls weitere Hilfsstoffe wie Coemulgatoren, Konsistenzgeber, Verdicker, Stabilisatoren, Polyole, Hydrotrope, Konservierungsmittel, Wirkstoffe, UV-Filter sowie Puder- und Füllstoffe.

So enthalten erfindungsgemäße Emulsionen O/W-Emulsionen mindestens einen Emulgator mit einem HLB Wert > 7 und gegebenenfalls einen Coemulgator. Diese Emulgatoren können vorteilhaft gewählt werden aus der Gruppe der nichtionischen oder anionischen Emulgatoren.

Unter den nichtionischen Emulgatoren befinden sich vorzugsweise
a) Partialfettsäureester und Fettsäureester mehrwertiger Alkohole und deren ethoxilierte Derivate (z. B. Glycerylmonostearate, Sorbitanstearate, Glyceryl Stearyl Citrate, Sucrose Stearate)
b) ethoxilierte Fettalkohole und Fettsäuren.

O/W-Emulgatoren, mit denen sich erfindungsgemäße O/W-Emulsionen herstellen lassen, sind vor allem nichtionische oder anionische Emulgatoren. Vorteilhafte nichtionische O/W-Emulgatoren sind ethoxylierte Fettalkohole oder Fettsäuren, bevorzugt PEG-100-Stearat, PEG-40-Stearat, Ceteareth-20, Ceteth-20, Steareth-20, Ceteareth-12, Ceteth-12, Steareth-12, sowie Ester aus Mono-, Oligo- oder Polysacchariden mit Fettsäuren, bevorzugt Cetearylglucosid, Methylglucosedistearat.

Vorteilhafte anionische Emulgatoren sind Seifen (z. B. Natrium- oder TriethanolaminSalze der Stearin- oder Palmitinsäure) sowie Ester der Zitronensäure wie Glycerylstearatcitrat.

Als geeignete Coemulgatoren für die erfindungsgemäßen O/W-Emulsionen können Fettalkohole mit 8 bis 30 Kohlenstoffatomen, Monoglycerinester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, Propylenglycolester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, sowie Sorbitanester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, eingesetzt werden.

Besonders vorteilhafte Coemulgatoren sind Glycerylmonostearat, Glycerylmonooleat, Diglycerylmonostearat, Sorbitanmonoisostearat, Saccharosedistearat, Cetylalkohol, Stearylalkohol, Behenylalkohol, Isobehenylalkohol und Polyethylenglycol(2)stearylether (Steareth-2).

Es kann im Sinn der vorliegenden Erfindung vorteilhaft sein, weitere Emulgatoren zu verwenden. So kann beispielsweise die Wasserfestigkeit der erfindungsgemäßen Zubereitungen erhöht werden. Geeignete Emulgatoren sind z. B. Alkylmethiconcopolyole und Alkyl-Dimethiconcopolyole, insbesondere Cetyldimethiconcopolyol, Laurylmethiconcopolyol, W/O-Emulgatoren wie Sorbitanstearat, Glycerylstearat, Glycerolstearat, Sorbitanoleat, Lecithin, Glycerylisostearat, Polyglyceryl-3-oleat, Polyglyceryl-3-diisostearat, PEG-7-hydriertes Ricinusöl, Polyglyceryl-4-isostearat, Acrylat/ C₁₀₋₃₀-Alkylacrylat-Crosspolymer, Sorbitanisostearat, Poloxamer 101, Polyglyceryl-2-dipolyhydroxy-stearat, Polyglyceryl-3-diisostearat, Polyglyceryl-4-dipolyhydroxystearat, PEG-30-dipoly-hydroxystearat, Düsostearoylpolyglyceryl-3-düsostearat, Glycoldistearat und Polyglyceryl-3-dipolyhydroxystearat.

Den erfindungsgemäßen O/W-Emulsionen können hydrophile Gelbildner beispielsweise zur Stabilisierung zugesetzt werden. Vorteilhafte Gelbildner für derartige Zubereitungen sind z. B. Copolymere aus C₁₀₋₃₀-Alkylacrylaten und einem oder mehreren Monomeren der Acrylsäure, der Methacrylsäure oder deren Ester. Die INCI-Bezeichnung für solche Verbindungen ist "Acrylates/C 10-30 Alkyl Acrylate Crosspolymer". Insbesondere vorteilhaft sind Pemulen® TR 1, Pemulen® TR 2 und Pemulen® TRZ von der Fa. Noveon.

Auch Carbomere sind vorteilhafte Gelbildner für derartige Zubereitungen. Carbomere sind Polymere der Acrylsäure, insbesondere Acrylat-Alkylacrylat-Copolymere. Vorteilhafte Carbomere sind beispielsweise Carbopol 980, Carbopol 981, Carbopol 1342, Carbopol 1382, Carbopol 2984 und Carbopol 5984 sowie Carbopol ETD 2020, Carbopol ETD 2050 und Carbopol Ultrez 10 der Fa. Noveon. Weitere vorteilhafte Gelbildner für derartige Zubereitungen sind Xanthan Gummi, Cellulose Derivate oder Johannisbrotkernmehl sowie Acrylamidmethylpropylsulfonsäurepolymere (so genannte AMPS-Polymere) insbesondere hydrophobisierte Acrylamidomethylpropylsulfonsäure-Polymere, die am AMPS-Grundgerüst über PEG-Ketten (mit 2 bis 200, vorteilhaft 5 bis 25 Ethoxyeinheiten) gebundene Alkylketten tragen, wobei die Kettenlänge im Bereich zwischen C₆ und C₂₂ liegt.

Zur Verbesserung des Hautgefühls und der Stabilität sowie zur Einstellung der Viskosität können beispielsweise Verdicker und Konsistenzgeber zugesetzt werden. Besonders vorteilhafte Konsistenzgeber sind hierbei Glycerylstearat, -palmitat oder -oleat sowie Fettalkohole, vor allem unverzweigte gesättigte mit 10 bis 24 C-Atomen. Auch Wachse können als Konsistenzgeber eingesetzt werden.

Weiterhin vorteilhaft können Filmbildner enthalten sein, welche beispielsweise eine gleichmäßigere Verteilung der Pigmente auf der Haut und eine Verbesserung der Transferresistenz der auf der Haut aufgetragenen Zubereitung bewirken. Besonders vorteilhafte Filmbildner sind hierbei VP Eicosene Copolymer, VP Hexadecene Copolymer, Sucrose Acetate Isobutyrate sowie Trimethylsiloxysilicate.

Eine vorteilhafte erfindungsgemäße O/W-Emulsion ist beispielsweise eine O/W-Maskara. Es handelt sich hierbei bevorzugt um ein mit Triethanolamin verseiftes Stearatsystem, zu dem noch weitere Emulgatoren gegeben werden können, mit hohen Mengen an Wachsen, Pigmenten und Filmbildnern. Außer Triethanolamin kann auch Aminomethylpropanol als Verseifungsmittel für die Stearinsäure verwendet werden. Vorzugsweise liegt die Einsatzkonzentration von Triethanolamin-Stearat bei 5 bis 8 Gew.-%, die der Pigmente bei 8 bis 12 Gew.-%, Wachse von 8 bis 15 Gew.-%. Als Filmbildner können sowohl hydrophile als auch lipophile Filmbildner eingesetzt werden. Weiterhin ist der Einsatz von Verdickern wie z. B. Hydroxyethylcellulose sinnvoll.

Die Wachse können aus der Gruppe der Bienenwachse und ihrer Teilfraktionen sowie der Bienenwachsderivate, der Gruppe der paraffinischen Kohlenwasserstoffwachse wie mikrokristalline Wachse und Ozokerite sowie aus der Gruppe der homopolymeren Polyethylene gewählt werden. Ebenfalls vorteilhaft können natürliche Wachse wie Reiswachs, Candellilawachs, Carnaubawachs, Japanwachs oder Schellackwachs eingesetzt werden.

Zur Herstellung von erfindungsgemäßen O/W-Emulsionen wird zunächst eine erfindungsgemäße Pigment-Dispersion (Pigmentphase) aus Pigmenten und dem polaren Öl wie oben beschrieben hergestellt. Diese Dispersion kann hierbei zusätzlich weitere Bestandteile der Ölphase enthalten. Die Pigmentphase wird vor der Phasenvereinigung (Wasser - Öl) der Ölphase zugesetzt.

Weitere erfindungsgemäße Zubereitungen können W/O- bzw. Wasser-in-Silikonöl- (W/S-) Emulsionen sein. Vorteilhaft im Sinn der Erfindung sind dabei W/O- oder W/S-Emulsionen, die
● ein oder mehrere Silikonemulgatoren (W/S) mit einem HLB-Wert ≤ 8 oder ein oder W/O-Emulgatoren mit einem HLB-Wert < 7 oder eine Mischung dieser Emulgatoren und gegebenenfalls
● ein oder mehrere O/W-Emulgatoren mit einem HLB-Wert > 10 enthalten.

Die erfindungsgemäßen W/O-Emulsionen enthalten vorteilhaft mindestens 20 Gew.-% an Ölphase, wobei die Ölphase Silikonöle enthalten oder ganz aus Silikonölen bestehen kann (W/S-Emulsionen). Weiterhin können die erfindungsgemäßen W/O- oder W/S-Emulsionen Feststoffe in einem Bereich von 5 bis 25 Gew.-% enthalten, wobei sich die Feststoffphase bevorzugt aus Pigmenten und Füllstoffen zusammen setzt.

Die Silikonemulgatoren können vorteilhaft aus der Gruppe enthaltend Alkyldimethiconcopolyole wie z. B. Cetyl PEG/PPG 10/1 Dimethiconcopolyol (ABIL® EM 90 der Fa. Goldschmidt AG) oder Lauryl PEG/PPG- 18/18 Dimethicone (Dow Corning® 5200 Formulation Aid der Fa. Dow Corning Ltd.) und Dimethiconecopolyole wie z. B. PEG-10 Dimethicone (KF-6017 der Fa. Shin Etsu), PEG/PPG- 18/18 Dimethicone (Dow Corning Formulation aid 5225C der Fa. Dow Corning Ltd.) oder PEG/PPG-19/19 Dimethicone (Dow Corning BY-11 030 der Fa. Dow Corning Ltd.) gewählt werden.

Die W/O-Emulgatoren mit einem HLB-Wert < 7 können vorteilhaft aus der Gruppe enthaltend Sorbitanstearat, Sorbitanoleat, Glycerylisostearat, Polyglyceryl-3-oleat, Pentaerythrithylisostearat, Methylglucosedioleat, PEG-7-hydriertes Ricinusöl, Polyglyceryl-4-isostearat, Hexyllaurat, Sorbitanisostearat, Polyglyceryl-2-dipolyhydroxystearat, Polyglyceryl-3-Diisostearat, PEG-30-dipolyhydroxystearat, Diisostearoylpolyglyceryl-3-diisostearat, Polyglyceryl-3-dipolyhydroxystearat, Polyglyceryl-4-dipolyhydroxystearat, Polyglyceryl-3-dioleat und Wollwachsalkohol (Eucerit) gewählt werden.

Die O/W-Emulgatoren mit einem HLB-Wert > 10 können vorteilhaft aus der Gruppe enthaltend Lecithin, Trilaureth-4-phosphat, Polysorbate-20, Polysorbate-60, PEG-22-Dodecylglycol Copolymer, Sucrosestearat und Sucroselaurat gewählt werden.

Zur Stabilisierung der erfindungsgemäßen Emulsionen gegen Sedimentierung oder Flockung der Wassertröpfchen kann es von Vorteil sein, darüber hinaus noch Ölverdicker hinzuzusetzen. Besonders geeignet sind hierfür Aerosil (ggf. modifizierte pyrogene Kieselsäure), Silikate wie Bentonit, Hectorit oder Montmorillonit (zur Optimierung der Eigenschaften ggf. obertlächenbehandelt) sowie Magnesium- oder Aluminiumstearat.

Weiterhin vorteilhaft kann der Einsatz von Wachsen sein, insbesondere von silikonmodifizierten Wachsen wie z. B. Cetearyl Methicone, Siliconyl Beeswax oder C30-45 Alkyl Dimethicone.

Ebenso können der Ölphase der erfindungsgemäßen Emulsionen weitere Silikonöle, silikonmodifizierte Öle zugesetzt werden. Als vorteilhafte Silikonöle können Dimethicone, Phenyl Trimethicone oder Bis-Phenylpropyl Dimethicone eingesetzt werden. Vorteilhafte silikonmodifizierte Öle sind z. B. Methyl Ester Siloxane (GE Bayer SF 1318 der Fa. Bayer) oder Phenylpropyl Dimethylsiloxysilicat (GE Bayer Silshine 151 der Fa. Bayer).

Die erfindungsgemäße Emulsion kann zusätzlich Filmbildner enthalten, um z. B. eine gleichmäßigere Verteilung der Pigmente auf der Haut zu erhalten und die Transferresistenz der auf die Haut aufgetragenen Zubereitung zu verbessern.

Vorteilhafte Filmbildner sind z. B. Trimethylsiloxysilicate (z. B. SR 1000 GE der Fa. Bayer), Silikon Acrylate Copolymere (z. B. TIB4-200 der Fa. Dow Corning oder KP- 561 der Fa. Shin Etsu) oder Trimethyl Pentaphenyl Trisiloxane (Dow Corning 555 Cosmetic Fluid der Fa. Dow Corning Ltd.).

Darüber hinaus kann es von Vorteil sein, der Rezeptur nicht-partikuläre sensorische Additive zuzusetzen, welche aus der Gruppe der Dimethiconole (z. B. Dow Corning 1503 Fluid der Fa. Dow Corning Ltd.), der Siliconcopolymere (z. B. Divinyldimethicone/Dimethicone Copolymer, Dow Corning HMW 2220 der Fa. Dow Corning Ltd.) oder der Siliconelastomere (z. B. Dimethicone Crosspolymer, Dow Corning 9040 Silicone Elastomer Blend der Fa. Dow Corning Ltd.) gewählt werden können.

Zur Herstellung von erfindungsgemäßen W/O- und W/Si-Emulsionen wird zunächst eine erfindungsgemäße Pigment-Dispersion (Pigmentphase) aus Pigmenten und dem polaren Öl wie oben beschrieben hergestellt. Diese Dispersion kann hierbei zusätzlich weitere Bestandteile der Ölphase enthalten. Die Pigmentphase wird vor der Phasenvereinigung (Wasser - Öl) der Ölphase zugesetzt.

Weitere Hilfsstoffe, die sowohl in W/O- als auch in O/W-Emulsionen eingesetzt werden können, sind beispielsweise Konservierungsmittel, Puder- und Füllstoffe, Hydrotrope und Wirkstoffe.

Geeignete Konservierungsmittel für Emulsionen sind Parabene, Phenoxyethanol, lodopropinylbutylcarbamat, Sorbinsäure, DMDM Hydantoin, Diazolidinylharnstoff, Sorbinsäure (vor allem bei pH-Werten kleiner 5) und Benzylalkohol. Es ist bekannt, dass Mischungen von Konservierungsmitteln synergistische Effekte zeigen, weshalb i. d. R. verschiedene Kombinationen mikrobiologischen Belastungstests unterzogen werden. Es kann darüber hinaus vorteilhaft sein, die Konservierungsmittel in anderen Stoffen gelöst einzusetzen, vor allem Polyole finden hier vorteilhaft Verwendung.

Geeignete Puder- und Füllstoffe sind insbesondere Stärke und Stärkederivate (z. B. Aluminum- oder Natrium-Stärke-Octenylsuccinat, Distärkephosphat), Talkum, Glimmer (Mica), Nylon (z. B. Nylon-12 oder Nylon-6/12), pyrogene Kieselsäuren, Kaolin oder partikuläres Polyethylen zum Einsatz. Die Teilchengröße der Rohstoffe liegt bevorzugt zwischen 1 und 30 µm und ihr Brechungsindex ist vorteilhaft zwischen 1 und 5.

Geeignete Hydrotrope sind aufgrund ihrer guten Hautpflegeeigenschaften insbesondere wasserlösliche organische Verbindungen wie z. B. Ethanol, Isopropanol, Harnstoff, Ethylhexyloxyglycerin, Methylpropandiol, Glycerin oder Alkandiole mit 2 bis 10 Kohlenstoffatomen wie Propylenglykol, Butylenglykol usw.

Es sind zahlreiche Wirkstoffe bekannt, die auch in der vorliegenden Erfindung vorteilhaft zum Einsatz kommen können. Besonders geeignet sind Vitamine (A, B, C, E und ihre Derivate und Provitamine), Antioxidantien (Flavonoide, Isoflavonoide und ihre Derivate). Auch Komplexbildner wie EDTA und Iminodibernsteinsäure können vorteilhaft im Sinne der vorliegenden Erfindung eingesetzt werden.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele

### 1. Lippenstifte

**Beispiel 1a: Pflegende Lippenstifte**

| **Inhaltsstoff (INCI-Bezeichnung)** | **Anteil in Gew.-%** | | | |
|---|---|---|---|---|
| | **1** | **2** | **3** | **4** |
| Rizinusöl | ad 100 | | 6,0 | 3,0 |
| Caprylic-/Capric Triglycerides | 20,0 | 20,0 | ad 100 | 3,0 |
| Pentaerythrityl Tetraisistearate | | 5,0 | | |
| Macadamiaöl | | | 2,0 | |
| Octyldodecanol | 20,0 | 5,0 | 6,0 | 6,0 |
| Hydrogenated Polyisobutene (z. B. Parleam Type 4, Rossow Cosmétiques) | | | 10,0 | 1,0 |
| Polyisobutene | | 2,0 | 3,0 | |
| Isopropylpalmitat | | 3,5 | 6,0 | 2,0 |
| Jojobaöl | 6,0 | | | 1,0 |
| Lanolin ÖI | 5,0 | | 9,0 | 1,0 |
| PEG-45/ Dodecyl Glycol Copolymer | | | | 2,0 |
| Polyglyceryl-3 Diisostearat | 3,7 | 3,5 | | 2,4 |
| Bis-Diglyceryl Polyacyladipat-2 | | 6,0 | | |
| Cetearyl Alkohol | 6,0 | 0,5 | | |
| Cetylpalmitat | 7,5 | | | 1,0 |
| C20-40 Alkyl Stearat | 16,0 | | 3,0 | 8,0 |
| Carnaubawachs | | 5,0 | 4,0 | 2,0 |
| Candelillawachs | | 3,0 | 4,0 | |
| Bienenwachs | 6,0 | 2,0 | | |
| Microcristallines Wachs | | 8,0 | 4,0 | 8,0 |
| PVP / Eicosen Copolymer | | | 0,5 | 0,2 |
| 4-Methylbenzyliden Campher | | | | 5,0 |
| Octyl Methoxycinnamat | 3,0 | 3,0 | 3,0 | 2,5 |
| Ethylhexyl Triazon | | | 2,0 | 6,0 |
| Octocrylen | | 3,0 | | |

**Fortsetzung, Beispiel 1a**

| **Inhaltsstoff (INCI-Bezeichnung)** | **Anteil in Gew.-%** | | | |
|---|---|---|---|---|
| | **1** | **2** | **3** | **4** |
| Butyl Methoxydibenzoylmethan | 2,0 | | | 0,8 |
| Lauroyl Lysin | 0,5 | | 0,6 | |
| Chinacridon-Pigment Dispersion, 40%ig | 0,5 | 2,6 | 4,2 | 6,0 |
| Titandioxid | | 0,6 | 1,0 | 2,0 |
| Eisenoxide | | 2,4 | 2,0 | 1,0 |
| Effektpigmente | | 6,0 | 3,5 | |
| Glimmer | | | 4,0 | |
| Zitronensäure | 0,05 | | | |
| Glycerin | | | | 10 |
| Tocopheryl Acetate | 0,5 | | 0,5 | 1,0 |
| Ascorbyl Palmitate | | 0,2 | 0,3 | |
| Ubichinon | | 0,5 | | |
| Parfum, Konservierungsmittel, BHT, Neutralisationsmittel, Sequestriermittel | q. s. | q. s. | q. s. | q. s. |
| Wasser | 2,5 | | | ad 100 |

**Beispiel 1b: Langhaltende Lippenstifte**

| **Inhaltsstoff (INCI-Bezeichnung)** | **Anteil in Gew.-%** | | | | |
|---|---|---|---|---|---|
| | **1** | **2** | **3** | **4** | **5** |
| Mikrokristallines Wachs | 3,0 | 4,0 | | 2,5 | 8,0 |
| Polyethylenwachs | | 6,0 | 10,0 | 12,0 | |
| C30-C50 Fettalkohole | | | 10,0 | 6,0 | |
| Carnaubawachs | | | | | 2,0 |
| Candelillawachs | 12,0 | 2,0 | | 6,0 | 8,0 |
| Bienenwachs | 2,5 | | | | 1,0 |
| C20-40 Alkyl Stearat | | | | 1,5 | |
| Lanolinöl | | 3,0 | | 2,0 | |
| Bis-Diglyceryl Polyacyladipat-2 | | 5,0 | | | 6,0 |
| Polyglyceryl-3 Diisostearate | 10,0 | | | | |
| Cyclomethicone | 25,0 | | | 25,0 | 30,0 |
| Isododecan | | 50,0 | 45,0 | 10,0 | 5,0 |
| Jojobaöl | | | | | 3,0 |
| Octyldodecanol | ad 100 | | ad 100 | ad 100 | |
| Polybutene | 5,0 | | 3,0 | | 2,0 |
| Caprylic/Capric Triglycerides | 12,0 | ad 100 | | | ad 100 |
| Pentaerythrityl Tetraisostearate | 10,0 | | | | 3,0 |
| Acrylates Copolymere | | | 8,6 | 6,0 | |
| Styrene Copolymere | | | 2,0 | | 2,0 |
| Trimethylsiloxysilicate | | 0,5 | | 2,6 | 2,0 |
| PVP/Hexadecen Copolymer | 0,5 | | | 0,3 | |
| Silica Dimethyl Silylat (Aerosil R972) | | 0,5 | | 0,5 | 0,2 |
| Polymethylsilsesquioxan (Tospearl 2000B) | | | 3,0 | 3,0 | 3,0 |
| Disteardimonium Hectorite | | | 0,5 | | 0,5 |
| Propylen Carbonat | | | 0,12 | | 0,12 |
| Ethylhexyl Methoxycinnamat | 0,5 | 0,5 | | 2,0 | 0,5 |
| Butyl Methoxydibenzoylmethan | 0,2 | 0,2 | | | 0,2 |
| Bis-Ethylhexyloxyphenol Methoxyphenyl Triazin | | 2,0 | | 0,25 | |
| Mikronisiertes Titandioxid (Eusolex T 2000) | | 2,0 | | | 2,0 |
| Goniochromatische Pigmente | 5,0 | | | 6,0 | 8,0 |
| Metallpigmente | | | 2,0 | | 2,0 |
| Interferenz Pigmente | | 4,0 | 3,0 | 3,0 | |
| Titandioxid Cl 77891 | 1,0 | 3,8 | 2,0 | | |
| Eisenoxide Cl 77491, 77492, 77499 | 0,6 | 2,2 | | | |
| CI 15850 | | | 1,9 | | |
| CI 42090 | 0,3 | | | 0,6 | |
| CI 19140 | | 0,6 | | | |
| CI 15985 | | | 1,0 | | |
| CI 45380 | | | | 0,6 | |
| CI 73360 | | 1,5 | | | |
| Chinacridon-Pigment | 6,3 | 0,6 | 2,5 | 2,5 | 5,0 |
| Tocopheryl Acetat | 1,0 | 2,0 | 0,5 | 1,5 | 1,0 |
| Konservierungsmittel, BHT, Parfum, Aroma | q. s. | q. s. | q. s. | q. s. | q. s. |

**Beispiel 1c: Hochglänzende Lippenstifte**

| **Inhaltsstoff (INCI-Bezeichnung)** | **Anteil in Gew.-%** | | | | |
|---|---|---|---|---|---|
| | **1** | **2** | **3** | **4** | **5** |
| Mikrokristallines Wachs | 5,0 | 8,0 | 3,5 | 2,0 | |
| Polyethylenwachs | 5,0 | 6,0 | 8,0 | 8,0 | 8,0 |
| C30-C50 Fettalkohole | | | 6,0 | 2,0 | 6,0 |
| Candelillawachs | 5,0 | 2,0 | | 2,0 | 2,0 |
| C20-40 Alkyl Stearat | | | | 0,5 | |
| Phenyltrimethicone | | 10,0 | | | 5,0 |
| Phenylpropyldimethylsiloxysilicate | 5,0 | | 10,0 | 15,0 | |
| Trimethyl Pentaphenyl Trisiloxane (DC Cosmetic Fluid555) | 5,0 | | 10,0 | | 5,0 |
| Methyl Ether Siloxane (GE SF 1318) | 5,0 | | | 2,0 | 10,0 |
| Triisocetylcitrate | 10,0 | | | | 5,0 |
| Diisosteraryl Malate | 5,0 | | 10,0 | 8,0 | 5,0 |
| Lanolinöl | | 9,0 | | | 3,0 |
| Cocoglycerides | ad 100 | | ad 100 | 5,0 | ad 100 |
| Rizinusöl | | ad 100 | | ad 100 | 2,0 |
| Cetyl Ricinoleate | | 6,0 | | | 0,8 |
| Avocadoöl | 10,0 | 5,0 | | | |
| Isopropyl Palmitate | | | 6,0 | | 5,0 |
| Pentaerythrityl Tetraisostearat | | 2,0 | 10,0 | 5,0 | |
| Polyisobuten | | 5,0 | | 5,0 | |
| hydriertes Polydecen | | 13,0 | | 3,0 | |
| Polyglyceryl-3 Diisostearate | | | | 0,5 | |
| Ethylhexyl Methoxycinnamat | 3,0 | | 4,5 | | 8,5 |
| Bis-Ethylhexyloxyphenol methoxyphenyl Triazin | 0,5 | 3,0 | 2,5 | 1,0 | |
| Butyl Methoxydibenzoylmethan | | | | | 2,0 |
| Ethylhexyl Triazon | | 2,0 | | | |
| Octocrylen | 6,0 | | 3,0 | 2,0 | |
| Diethylhexyl Butamido Triazon | | | 1,5 | | |
| Methylen Bis-Benzotriazolyl Tetramethylbutylphenol | 3,5 | 0,75 | | | 2,0 |
| Diethylhexyl-2,6-naphthalat | | | | | 5,5 |
| Disteardimonium Hectorite | | 0,5 | | | |
| Propylen Carbonat | | 0,12 | | | |
| Silica Dimethyl Silylate (Aerosil R972) | | | | | 0,3 |
| Glimmer | | | | 5,0 | |
| Calcium Aluminium Borosilikat (z. B. Rona Star Nobel Sparks, Merck) | | | 10,0 | 6,0 | 3,0 |
| Interferenz Pigmente | 10,0 | 6,8 | 0,6 | | |
| Chinacridon | 2,8 | 0,6 | 6,0 | 3,6 | 1,2 |
| Titandioxid Cl 77891 | | 2,0 | | 2,0 | 2,0 |
| Eisenoxide Cl 77491, 77492, 77499 | | | 0,6 | 1,5 | 2,0 |
| Cl 15850 | 1,3 | 3,2 | | 1,5 | 0,5 |
| Cl 42090 | | | 0,2 | | |
| Cl 17200 | | 0,3 | | 0,75 | |
| Cl 45380 | 0,1 | | | | 0,05 |
| Cl 15985 | | | | | |
| Cl 73360 | | 1,0 | | | |
| Cl 45410 | | | | 0,5 | |
| Tocopheryl Acetate | 0,5 | | 1,0 | 1,5 | |
| Parfüm, Konservierungsmittel, Filmbildner, Antioxidantien, etc. | q. s. | q. s. | q. s. | q. s. | q. s. |

**Beispiel 1d: Lippengloss**

| **Inhaltsstoff (INCI-Bezeichnung)** | **Anteil in Gew.-%** | | |
|---|---|---|---|
| | **Gloss** | **Glossy roll on** | **Gloss Lack** |
| Polyisobuten | ad 100 | ad 100 | |
| Polybuten | | | ad 100 |
| Pentaerythrityl Tetraisostearate | 12,0 | 5,0 | 10,0 |
| Hydrogenated Polydecene | 3,0 | 30,0 | |
| Diisosteraryl Malate | 5,0 | 2,5 | |
| Hydrogenated Polyisobutene | 1,0 | | |
| Myristyl Lactate | 5,0 | | |
| Avocadoöl | 5,0 | | |
| Polyethylenwachs | 1,0 | | 1,0 |
| C30-C50 Fettalkohole | | 0,3 | |
| Trimethylsiloxysilicate | | | 0,3 |
| Triisocetylcitrate | 10,0 | | |
| Octyldodecanol | | | 3,0 |
| Polyglyceryl-2 Tetraisostearate | | | 4,0 |
| Cocoglycerides | | 2,0 | |
| Rizinusöl | | 2,0 | |
| Disteardimonium Hectorite | 0,5 | | 0,75 |
| Propylen Carbonat | 0,12 | | 0,18 |
| Silica Dimethyl Silylate (Aerosil R972) | 0,8 | | 0,6 |
| Calcium Aluminium Borosilikat (z. B. Rona Star Nobel Sparks, Merck) | | | 10,0 |
| Interferenz Pigmente | 10,0 | | |
| Titandioxid CI 77891 | | 2,0 | |
| Eisenoxide CI 77491, 77492, 77499 | | | 0,6 |
| CI 15850 | | 1,3 | |
| CI 15985 | | 0,6 | |
| CI 42090 | | | 0,4 |
| CI 17200 | 0,6 | | |
| Chinacridon | 2,5 | 1,2 | 3,0 |
| Tocopheryl Acetate | 0,5 | 0,5 | 1,0 |
| Parfüm, Konservierungsmittel, Filmbildner, Antioxidantien, etc. | q. s. | q. s. | q. s. |

**Beispiel 1e: Lippenkonturenstift**

| **Inhaltsstoff (INCI-Bezeichnung)** | **Anteil in Gew.-%** | |
|---|---|---|
| Cyclomethicone | 45 | 34,2 |
| Trimethylsiloxysilicate | 12,5 | 10 |
| Polyethylene | 7 | 10,5 |
| Cera Alba | 3 | 6,5 |
| Di-PPG-3 Myristyl Ether Adipate | 3 | 6 |
| Polybutene | 3,5 | 4,5 |
| Cl 15850 | 2,5 | 3 |
| Polyethylene | 2,5 | 3,5 |
| Sucrose Tetrastearate Triacetate | 1,9 | 2,5 |
| Isodecyl Neopentanoate (44,5%) | 2,5 | 2 |
| Stearalkonium Bentonite (7,5%) | 0,5 | 1,5 |
| Propylene Carbonate (3%) | 0,03 | 0,5 |
| Dimethicone/Vinyl Dimethicone Crosspolymer (0,5%) | 0,05 | 0,4 |
| Mica | 1,9 | 2 |
| Pentaerythrityl Tetrabehenate | 0,8 | 1,5 |
| CI 42090 | 1,6 | 1 |
| Boron Nitride | 0,9 | 0,5 |
| Eisenoxide Cl 77491, 77492, 77499 | 1,5 | 8,5 |
| Titandioxid Cl 77891 | 1,5 | 0,5 |
| Chinacridon | 8,5 | 1,5 |
| Tocopherol | 0,5 | 0,25 |
| Ascorbyl Palmitate | 0,02 | 0,05 |
| Parfüm, Konservierungsmittel, ,Antioxidantien, etc. | q.s | q.s. |

### 2. Blush

**Beispiel 2a: Gegossenes Blush**

| **Inhaltsstoff (INCI-Bezeichnung)** | **Anteil in Gew.-%** |
|---|---|
| Caprylic / Capric Triglycerides | ad 100 |
| Cera Microcristallina | 3,5 |
| Bienenwachs | 4,0 |
| C20-40 Alkyl Stearat | 6,0 |
| Cetyl Plamitate | 2,5 |
| Isononyl Isononanoate | 6,0 |
| Lauroyl Lysin | 1,0 |
| Nylon | 4,0 |
| Interferenzpigmente | 6,0 |
| Titandioxid CI 77891 | 2,0 |
| Eisenoxide CI 77492, 77499 | 8,0 |
| Chinacridon | 2,0 |
| Cl 15850 | 0,3 |
| PVP/Eicosen Copolymer | 2,0 |
| Tocopheryl Acetate | 1,0 |
| Parfüm, Konservierungsmittel, Antioxidantien, etc. | q. s. |

### 3. Emulsionen

**Beispiel 3a: Emulsionen**

| **Inhaltsstoff (INCI-Bezeichnung)** | **Anteil in Gew.-%** | | |
|---|---|---|---|
| | **Emulsions-blush** | **Foundation** | **Getönte Tagescreme** |
| Stearic Acid | 1,5 | 2,0 | |
| Glycerinmonostearat | 2,0 | 1,5 | |
| PEG-100 Stearate | 1,2 | 0,6 | |
| Glyceryl Stearat Citrat | | | 3,5 |
| Octyldodecanol | 7,00 | | 3,0 |
| Cyclomethicone | 6,0 | 12,0 | 3,0 |
| Ceatearyl Alcohol | | | 1,5 |
| Squalane | | | 2,0 |
| Diisostearyl Malate | 2,0 | | |
| Dimethicone / Vinyl Dimethicone Copolymer (z. B. Dow Corning HMW 2220) | 1,00 | | |
| Pentaerythrithyl Tetracocoate | 2,00 | | |
| Shea Butter | | | 5,0 |
| Carbomer | | | 0,6 |
| Xanthan Gum | 0,3 | 0,2 | |
| Magnesium Aluminium Silikate (z. B. Veegum) | 1,0 | 0,8 | |
| Glycerin | 5,0 | 3,0 | 10,0 |
| Chinacridon | 1,5 | 0,6 | 0,3 |
| Glimmer | 1,0 | | |
| Interferenzpigmente | | | 3,0 |
| Eisenoxide | 3,0 | 3,6 | |
| Titandioxid | 2,5 | 5,0 | |
| Talkum | 5,0 | 2,0 | |
| Octyl Methoxy Cinnamat | | | 2,0 |
| mikronisiertes Titandioxid | | | 2,0 |
| Butyl Methoxy Dibenzoylmethan | | | 0,5 |
| Tocopherolacetat | 1,0 | | 1,0 |
| Antioxidantien, Konservierungsmittel, Neutralisationsmittel, Parfum etc. | q. s. | q. s. | q. s. |
| Wasser | ad 100 | ad 100 | ad 100 |

## Patentansprüche

1. Kosmetische Zubereitung zur dekorativen Anwendung auf der Haut, **dadurch gekennzeichnet, dass** sie Chinacridon-Pigmente und ein Öl, welches eine Grenzflächenspannung gegenüber Wasser kleiner als 30 mN/m aufweist, enthält.

2. Kosmetische Zubereitung nach Anspruch 1, **dadurch gekennzeichnet, dass** das ÖI eine Grenzflächenspannung gegenüber Wasser kleiner als 25 mN/m aufweist.

3. Kosmetische Zubereitung nach Anspruch 1, **dadurch gekennzeichnet, dass** das Öl eine Grenzflächenspannung gegenüber Wasser kleiner als 20 mN/m aufweist.

4. Kosmetische Zubereitung nach einem der vorhergehenden Ansprüche, **dadurch gekennzeichnet, dass** das ÖI eine Viskosität größer als 2 mPas aufweist.

5. Kosmetische Zubereitung nach Anspruch 4, **dadurch gekennzeichnet, dass** das ÖI eine Viskosität größer als 10 mPas aufweist.

6. Kosmetische Zubereitung nach Anspruch 4, **dadurch gekennzeichnet, dass** das ÖI eine Viskosität größer als 20 mPas aufweist.

7. Kosmetische Zubereitung nach einem der vorhergehenden Ansprüche, **dadurch gekennzeichnet, dass** das ÖI ein aliphatischer Kohlenwasserstoff ist, welcher linear oder verzweigt und/oder gesättigt oder ungesättigt ist.

8. Kosmetische Zubereitung nach einem der vorhergehenden Ansprüche, **dadurch gekennzeichnet, dass** das Öl aus der Gruppe enthaltend natürliche und synthetische Triglyceride, natürliche und synthetische Ester, Alkohole und Ether ausgewählt ist.

9. Kosmetische Zubereitung nach einem der vorhergehenden Ansprüche, **dadurch gekennzeichnet, dass** das Öl aus der Gruppe enthaltend Ricinusöl, Capryl-/Caprinsäuretriglyceride, Octyldodecanol, Pentaerythrit-tri/tetraisostearat, Diisostearylmaleat, Trimethylolpropantriisostearat, Distearylfumarat, PPG-15-Stearylether und Lanolin ausgewählt ist.

10. Kosmetische Zubereitung nach einem der Ansprüche 1 bis 9, **dadurch gekennzeichnet, dass** die Zubereitung 0,01 bis 80 Gew.-% Chinacridon-Pigmente enthält.

11. Kosmetische Zubereitung nach Anspruch 10, **dadurch gekennzeichnet, dass** die Zubereitung 0,1 bis 60 Gew.-% Chinacridon-Pigmente enthält.

12. Kosmetische Zubereitung nach Anspruch 1 bis 11, **dadurch gekennzeichnet, dass** die Zubereitung weitere Öle enthält.

13. Kosmetische Zubereitung nach Anspruch 12, **dadurch gekennzeichnet, dass** die weiteren Öle aus der Gruppe enthaltend
aliphatische Kohlenwasserstoffe mit einer Kettenlänge von 6 bis 22 Kohlenstoffatomen,
Ester aus gesättigten und ungesättigten Carbonsäuren, die eine Kettenlänge von 3 bis 30 Kohlenstoffatomen aufweisen, und gesättigten und ungesättigten Alkoholen, die eine Kettenlänge von 2 bis 30 Kohlenstoffatomen und 1 bis 5 Hydroxygruppen aufweisen,
Ether aus gesättigten oder ungesättigten Alkoholen, die eine Kettenlänge von 2 bis 30 Kohlenstoffatomen und 1 bis 5 Hydroxygruppen aufweisen,
Ester der Kohlensäure mit Alkoholen, die eine Kettenlänge von 2 bis 30 Kohlenstoffatomen und 1 bis 5 Hydroxygruppen aufweisen, cyclische und lineare Silikonöle sowie
Ester aus aromatischen Carbonsäuren und gesättigten und ungesättigten Alkoholen, die eine Kettenlänge von 2 bis 30 Kohlenstoffatomen und 1 bis 5 Hydroxygruppen aufweisen, ausgewählt sind.

14. Kosmetische Zubereitung nach einem der Ansprüche 1 bis 13, **dadurch gekennzeichnet, dass** die Zubereitung weitere feste Bestandteile enthält.

15. Kosmetische Zubereitung nach Anspruch 14, **dadurch gekennzeichnet, dass** die festen Bestandteile Wachse sind.

16. Kosmetische Zubereitung nach Anspruch 15, **dadurch gekennzeichnet, dass** die Wachse aus der Gruppe bestehend aus Bienenwachsen, Teilfraktionen des Bienenwachs und Bienenwachsderivaten, mikrokristallinen Wachsen, Ceresin, Ozokeriten, Carnaubawachs, Candelillawachs, Reiswachs, Japanwachs, Schellackwachs sowie homopolymeren Polyethylenen ausgewählt sind.

17. Kosmetische Zubereitung nach Anspruch 16, **dadurch gekennzeichnet, dass** die Zubereitung eine bei Temperaturen zwischen 70 und 100°C gießbare Masse ist.

18. Kosmetische Zubereitung nach Anspruch 17, **dadurch gekennzeichnet, dass** die Zubereitung Stift ist.

19. Kosmetische Zubereitung nach einem der Ansprüche 1 bis 18, **dadurch gekennzeichnet, dass** die Zubereitung eine oder mehrere wässrige Phasen enthält.

20. Kosmetische Zubereitung nach Anspruch 19, **dadurch gekennzeichnet, dass** die Zubereitung eine Emulsion oder Hydrodispersion ist.

21. Kosmetische Zubereitung nach Anspruch 20, **dadurch gekennzeichnet, dass** die Emulsion eine O/W-, eine W/O-, eine O/W/O-, eine W/O/W- oder eine Feststoff-Emulsion ist.

22. Kosmetische Zubereitung nach Anspruch 21, **dadurch gekennzeichnet, dass** die Emulsion eine W/O-Emulsion ist, deren Ölphase Silikonöl enthält oder aus Silikonöl besteht.

23. Kosmetische Zubereitung nach einem der Ansprüche 12 bis 22, **dadurch gekennzeichnet, dass** die Zubereitung 0,01 bis 20 Gew.-%, bevorzugt 0,05 bis 15 Gew.-%, besonders bevorzugt 0,1 bis 12 Gew.-%, Chinacridon-Pigmente enthält.
